# EUROPEAN PATENT SPECIFICATION

(11) **EP 4 522 230 B1**
(45) Date of publication and mention of the grant of the patent: **22.07.2026**
(21) Application number: 23726387.6
(22) Date of filing: 12.05.2023
(51) Int. Cl.: A61L 2/10

(54) **SYSTEM FOR THE MAINTENANCE AND STERILIZATION OF EMULSIONS**
SYSTEM ZUR WARTUNG UND STERILISIERUNG VON EMULSIONEN
SYSTÈME DE MAINTENANCE ET DE STÉRILISATION DES ÉMULSIONS

(30) Priority: 13.05.2022 EP 22382465
(43) Date of publication of application: 19.03.2025
(73) Proprietor: Universidad del País Vasco/Euskal Herriko Unibertsitatea, 48940 Leioa, Vizcaya (ES)
(72) Inventor: PEREIRA NETO, Octavio Manuel, 48940 Leioa - Vizcaya (ES); LÓPEZ DE LACALLE MARCAIDE, Luis Norberto, 48940 Leioa - Vizcaya (ES); GONZÁLEZ BARRIO, Haizea, 48940 Leioa - Vizcaya (ES); FERNÁNDEZ VALDIVIELSO, Asier, 48940 Leioa - Vizcaya (ES); RODRÍGUEZ EZQUERRO, Adrián, 48940 Leioa - Vizcaya (ES); AYESTA REMENTERÍA, Izaro, 48940 Leioa - Vizcaya (ES)
(74) Representative: ABG Intellectual Property Law, S.L.
(86) International application number: PCT/EP2023/062829
(87) International publication number: WO 2023/218063

(56) References cited:
- CN-A- 108 623 079
- CN-A- 111 779 076
- CN-A- 113 453 792
- CN-U- 208 865 556

## Description

### TECHNICAL FIELD OF THE INVENTION

The present invention belongs to the field of cutting fluids used in machining processes. More in particular, the invention relates to sterilization systems used for the maintenance and sterilization of oil emulsions.

### BACKGROUND OF THE INVENTION

The purpose of machining metal parts is to provide said parts with the desired shape, size and functionality by eliminating excess material in a controlled manner. Within the different types of machining, the most frequent machining operations by material removal are: turning, milling, drilling, broaching, profiling, brushing and filing.

In order to increase the performance of the machining process, reduce the wear of the cutting tool, and taking into account the need to improve the surface integrity after the machining process, cutting fluids are used and applied, by means of a continuous jet or a spray, at the point of contact between the edge of the tool and the part to be machined. These fluids also help to remove metal scrap and particles from the cutting area to prevent the formation of metal residues on the tool and the part to be machined.

Mineral, synthetic or vegetable-based oil emulsions are mainly used as cooling/lubricating fluids, where the oil is in a concentration ranging between 5% and 40%. Oil emulsions are mixtures of base oil which additionally may comprise emulsifiers, antiwear additives, extreme pressure additives, antifoaming agents, corrosion inhibitors, bactericides, etc.

In order to avoid the degradation of oil emulsions caused by the proliferation of microorganisms and therefore increase their shelf life, various techniques have been developed.

An example used in the industry consists of introducing carbon dioxide (CO₂) in gaseous form into the oil emulsion tank, achieving values of sterilization up to 90% after 3 hours of application of CO₂. However, the use of high concentrations of CO₂ implies the risk of suffocation on the part of the operators since the CO₂ displaces the oxygen due to its molecular weight.

Likewise, there are processes that contemplate the use of biocides such as phenolic derivatives, formaldehydes, or aliphatic derivatives, among others, for the control and elimination of said microorganisms. However, these biocides pose a health risk to workers as they cause skin irritation, allergic reactions and respiratory problems.

In addition, there is a risk that, through the loss of oil emulsion produced by hydraulic leaks and cleaning processes, the biocide ends up in the sewage system, thus causing environmental hazards.

Document CN 208 865 556 U discloses a constant-temperature sterile disinfection emulsion shaking and mixing device.

Document CN 108 623 079 A discloses a mobile sewage treatment vehicle based on A2O process.

Document CN 111 779 076 A discloses a whole-process clean life water tank.

Document CN 113 453 792 A discloses an air bubble generation device.

### SUMMARY OF THE INVENTION

The present invention provides a solution for the aforementioned problems, by a system for the maintenance and sterilization of oil emulsions housed within a reservoir according to claim 1, and by a system for the maintenance and sterilization of oil emulsions housed within a reservoir according to claim 4. In the dependent claims, preferred embodiments of the invention are defined.

In a first inventive aspect, the invention provides a system for the maintenance and sterilization of oil emulsions housed within a reservoir, said reservoir comprising a lower base according to the direction of gravity and an opening opposite said lower base, the system comprising:
- a supporting structure configured to be arranged, at least partially, in an operating mode of the system, over the opening of the reservoir;
- at least one UV lamp arranged on the supporting structure; and
- at least one conduit configured to be connected to a source of compressed air and configured to be inserted, at least partially, in an operating mode of the system, inside the reservoir, wherein said conduit is configured to establish fluidic communication between the source of compressed air and the interior of the reservoir through at least one compressed air diffusion means provided in the conduit.

The present invention makes it possible to increase the shelf life of oil emulsions used as cutting fluid in machining processes by combining UV light and compressed air for an optimal maintenance of the emulsion, avoiding the oil emulsion from demulsifying, as well as for the sterilization of the oil emulsion, preventing the proliferation of microorganisms while the oil emulsion is stored in a reservoir.

In particular, the proliferation of microorganisms is the parameter with the greatest influence on the durability of oil emulsions since these microorganisms act by accelerating their degradation and decreasing their efficiency, as well as posing a risk to operators in charge of handling said oil emulsion.

The source of ultraviolet (UV) light, that is, the at least one UV lamp of the system of the invention, is directly responsible for the sterilization process, also known as UV disinfection or ultraviolet germicidal irradiation (UVGI), which works by breaking some chemical bonds and altering the structure of DNA, RNA and some proteins, rendering a microorganism unable to multiply.

In this sense, it shall be understood, when reference is made through the description to ultraviolet light, or UV light, that three types of light with different wavelengths are to be considered:
- UVA: long-wave ultraviolet rays. They have a wavelength between 400 and 315 nm and an energy per photon between 3.10 and 3.94 eV.
- UVB: medium-wave ultraviolet rays. They have a wavelength between 315 and 280 nm and an energy per photon between 3.94 and 4.43 eV.
- UVC: short-wave ultraviolet rays. They have a wavelength between 280 and 200 nm and an energy per photon between 4.43 and 6.2 eV.

There are also EUV, which are extreme ultraviolet rays, closer to X-rays than the rest of referred types of UV light. They have a wavelength between 121 and 10 nm and an energy per photon between 10.25 and 12.4 eV.

For ultraviolet sterilization, UVC rays are preferably used because they have enough energy to effectively kill microorganisms, but not so much as to be as dangerous as EUV or X-rays.

The at least one UV lamp is arranged on a portion of the supporting structure such that, in an operating mode of the system, according to which the supporting structure is located, at least partially, over the opening of the reservoir, the UV lamp faces downwards so as to illuminate the opening of said reservoir.

By virtue of said structure and configuration of elements, i.e., the supporting structure on which the UV lamp is installed being capable of being arranged over the opening of a reservoir, the system of the invention is provided with a versatile capability to be used with any reservoir to perform the disinfection and sterilization process, thus simplifying installation.

Furthermore, the at least one UV lamp is decoupled from the interior of the reservoir, which contributes to preventing potential harmful contacts between the oil emulsion and the ultraviolet light source, i.e., the UV lamp.

Advantageously, preventing the UV light sources from being installed directly in the reservoir which stores the oil emulsion permits avoiding contact between the UV light sources and the oil emulsion, which would cause the UV light sources to become dirty, which would lead to a reduction in their efficiency, as well as to an increase in the associated maintenance cost.

According to the invention, compressed air is supplied to the fluid stored inside the reservoir for generating turbulence within said fluid. In particular, in an operating mode of the system, a source of compressed air is in fluidic communication with the interior of the reservoir (i.e., with the oil emulsion stored within) by means of at least one conduit inserted, in an operating mode of the system, inside the reservoir, said conduit being provided, in turn, with compressed air diffusion means through which the compressed air exits the conduit and is discharged into the reservoir.

Turbulence is thus originated in the oil emulsion, by means of compressed air bubbles which oxygenate the oil emulsion and therefore prevent the proliferation of anaerobic microorganisms, which are among the main causes of oil emulsion degradation and odour generation. Additionally, the use of compressed air in the form of bubbles implies the use of an easily obtainable gas which, unlike pH regulators or biocides such as those currently used in the industry, is harmless.

Furthermore, and alike the at least one UV lamp, the elements responsible for originating turbulence within the oil emulsion are decoupled from the reservoir, allowing an easy introduction into, and extraction from, the interior of the reservoir which stores the oil emulsion. That is, advantageously, with the system of the present invention the oil emulsion is treated in situ in the reservoir which contains the oil emulsion, thus not requiring to be pumped into additional equipment in order to be subjected to the respective treatment.

In an embodiment, the at least one UV lamp comprises a plurality of UV lamps.

In an embodiment, the conduit establishing fluidic communication between the source of compressed air and the interior of the reservoir, through the compressed air diffusion means, is coupled along at least a portion of the supporting structure.

In an embodiment, the compressed air diffusion means comprise at least one porous block adapted to be arranged at the lower base of the reservoir.

Advantageously, injecting the compressed air bubbles from the bottom of the reservoir generates upward turbulence in the oil emulsion, recirculating the whole volume of oil emulsion and preventing said volume from demulsifying. Additionally, by virtue of this recirculation of the whole volume of oil emulsion stored within the reservoir, the process of sterilization, performed by the UV lamps illuminating the upper surface of the fluid, is improved, given that oil emulsions have a certain opacity and the UV light is partially blocked, not penetrating into the interior of the reservoir.

Therefore, the sterilization effectiveness of UV light is increased because the oil emulsion is in recirculation and therefore the radiation reaches the entire oil emulsion and is not limited only to the upper surface thereof when it is at rest.

In an embodiment, the compressed air diffusion means comprise a plurality of porous blocks adapted to be arranged at the lower base of the reservoir.

In an embodiment, the system comprises a reservoir configured to store an oil emulsion within it.

In an embodiment, the system comprises processing means. In an embodiment, the processing means are configured for controlling the operation of one or several elements of the system.

In an embodiment, the processing means are configured for turning on/off the UV lamp.

In an embodiment, the system comprises a valve configured to control the passage of compressed air through the conduit. In an embodiment, the processing means are configured for controlling the operation of the valve.

In an embodiment, the valve is a solenoid valve.

In an embodiment, the system comprises a pressure sensor configured to:
- determine the pressure of the compressed air in a section of the conduit;
- generate an electrical signal comprising information about the determined pressure; and
- send said electrical signal to the processing means,
wherein the processing means are configured to control the operation of the valve based on the value determined by the pressure sensor to maintain the pressure of the compressed air along the conduit within a predetermined range.

In an embodiment, the processing means are configured to control the operation of the valve, based on the value of the pressure determined by the pressure sensor, to maintain the pressure of the compressed air along the conduit within the range of 4 - 8 bar.

In an embodiment, the processing means are programmed with a timer function for performing the following steps:
a) turning on the UV lamp and controlling the valve to prevent the passage of compressed air through the conduit into the reservoir until a predetermined time interval t₁ has elapsed; and
b) turning off the UV lamp and controlling the valve to allow the passage of compressed air through the conduit into the reservoir until a predetermined time interval t₂ has elapsed;
and wherein the processing means are further programmed to perform steps a) and b) intermittently.

In an embodiment, t₁ = t₂,
In an embodiment, the processing means are programmed with a timer function for performing the following sequence of instructions:
c) turning on the UV lamp and controlling the valve to allow the passage of compressed air through the conduit into the reservoir until a predetermined time interval t₃ has elapsed; and
d) turning off the UV lamp and controlling the valve to prevent the passage of compressed air through the conduit into the reservoir until a predetermined time interval t₄ has elapsed;
and wherein the processing means are further programmed to perform step c) and d) intermittently.

In an embodiment, t₃ = t₄,
In an embodiment, the processing means are programmed with a timer function for performing the following instruction:
e) turning off the UV lamp and controlling the valve to prevent the passage of compressed air through the conduit into the reservoir when a predetermined time interval t₅, during which the system has been operating, has elapsed.

Since UV sterilization uses UV energy to destroy biomolecules, its effectiveness depends on the total energy applied, which is affected by the exposure time. The implementation of different programs by the processing means:
- activating at intermittent intervals turbulence generation and UV exposure, and/or
- intercalating periods of combined action of both functions,
makes it possible to automate the operation of the system and ensure the sustained maintenance over time of the optimum conditions of the emulsion, even without the presence or intervention of operators.

In one embodiment, the supporting structure comprises at least one extendable or telescoping portion, which allows increasing a relative dimension of the supporting structure with respect to a reservoir comprising an oil emulsion intended to be sterilized.

In an embodiment, the supporting structure comprises at least one coupling portion configured to rest onto the contour of the opening of a reservoir such that, in an operating mode of the system, the UV lamp illuminates the opening of said reservoir. In an embodiment, the at least one UV lamp is arranged on a portion of the supporting structure at least partially surrounded by the at least one coupling portion.

By virtue of said structure and configuration of elements, i.e., the supporting structure comprising at least one coupling portion configured to rest onto the contour of the opening of a reservoir, the system of the invention is provided with a compact and robust structure and allows an easy installation.

In an embodiment, the supporting structure comprises a main body and a cantilever portion projecting from the main body, said cantilever portion being configured to be arranged, at least partially, in an operating mode of the system, over the opening of the reservoir, and wherein at least one UV lamp is arranged on a lower side of the cantilever portion facing downwards such that, in an operating mode of the system, the UV lamp illuminates the opening of said reservoir.

By virtue of said structure and configuration of elements, i.e., the supporting structure comprising a cantilever portion, on which the UV lamp is installed, the system of the invention is provided with a versatile capability to be used with any reservoir to perform the disinfection and sterilization process, thus simplifying installation.

In an embodiment, the conduit establishing fluidic communication between the source of compressed air and the interior of the reservoir, through the compressed air diffusion means, is coupled along at least a portion of the main body of the supporting structure, as well as along at least a portion of the cantilever portion.

In one embodiment, the main body comprises at least one extendable or telescoping portion, which allows increasing the relative height of the supporting structure with respect to a reservoir comprising an oil emulsion intended to be sterilized.

In one embodiment, the cantilever portion comprises at least one extendable or telescoping portion, which allows adjusting the relative position of the at least one UV lamp with respect to the opening of the reservoir.

In an embodiment, the supporting structure comprises a pedestal portion arranged on a lowermost portion of the main body, said pedestal portion comprising a substantially flat lower side, and being provided with a shape configured for withstanding the overturning moment caused by the main body and the cantilever portion of the supporting structure.

In an embodiment, the supporting structure comprises a plurality of wheels arranged on a lowermost side of the supporting structure.

In an embodiment, the system comprises a source of compressed air. In a more particular embodiment, the source of compressed air is housed in a box or case, arranged on the pedestal portion of the supporting structure of the system.

In an embodiment, the main body of the supporting structure projects substantially perpendicular from the pedestal portion.

In an embodiment, the cantilever portion projects essentially perpendicular from the main body.

In a second inventive aspect, the invention provides a method for sterilization of an oil emulsion housed within a reservoir, the method comprising the following steps:
- providing a system for the maintenance and sterilization of oil emulsions according to any embodiment of the first inventive aspect;
- arranging the system in relation to the reservoir such that the supporting structure is located, at least partially, over the opening of the reservoir;
- introducing inside the reservoir at least a portion of the conduit;
- activating at least one UV lamp arranged on a portion of the supporting structure for illuminating the opening of the reservoir;
- supplying compressed air from a source of compressed air to the oil emulsion inside the reservoir through at least one compressed air diffusion means provided in the conduit.

In a third inventive aspect, the invention provides an assembly for the maintenance and sterilization of oil emulsions, the assembly comprising:
- a reservoir configured for housing an oil emulsion, said reservoir comprising a lower base according to the direction of gravity and an opening opposite said lower base; and
- a system according to any embodiment of the first inventive aspect.

As for the term assembly, it shall be understood as a group of elements. Thus, regarding the third inventive aspect, the assembly shall be understood as the group comprising the system and the reservoir.

In an embodiment, the supporting structure is provided with suitable dimensions which permit the UV light sources bathe the upper fluid interface of an oil emulsion stored inside the reservoir.

In an embodiment, the supporting structure comprises at least one coupling portion configured to rest onto the contour of the opening of the reservoir such that, in an operating mode of the system, the at least one UV lamp illuminates the opening of the reservoir.

In an embodiment, the supporting structure comprises an extendable or telescoping portion that allows increasing a dimension of the supporting structure.

In an embodiment, the system comprises a supporting structure configured to be arranged, at least partially, in an operating mode of the system, over the opening of the reservoir, wherein the supporting structure comprises a main body and a cantilever portion projecting from the main body, said cantilever portion being configured to be arranged, at least partially, in an operating mode of the system, over the opening of the reservoir, and wherein at least one UV lamp is arranged on a lower side of the cantilever portion facing downwards such that, in an operating mode of the system, the UV lamp illuminates the opening of said reservoir.

In an embodiment, the main body and/or the cantilever portion of the supporting structure comprises at least one extendable or telescoping portion.

### DESCRIPTION OF THE DRAWINGS

These and other characteristics and advantages of the invention will become clearly understood in view of the detailed description of the invention which becomes apparent from preferred embodiments of the invention, given just as examples and not being limited thereto, with reference to the drawings.
- Figure 1: This figure shows a schematic representation of a system for the maintenance and sterilization of oil emulsions according to an embodiment of the present invention.
- Figure 2: This figure shows a schematic representation of system for the maintenance and sterilization of oil emulsions according to an embodiment of the present invention
- Figure 3: This figure shows a block diagram representing the way in which the elements of the system are related according to an embodiment of the present invention.

### DETAILED DESCRIPTION OF THE INVENTION

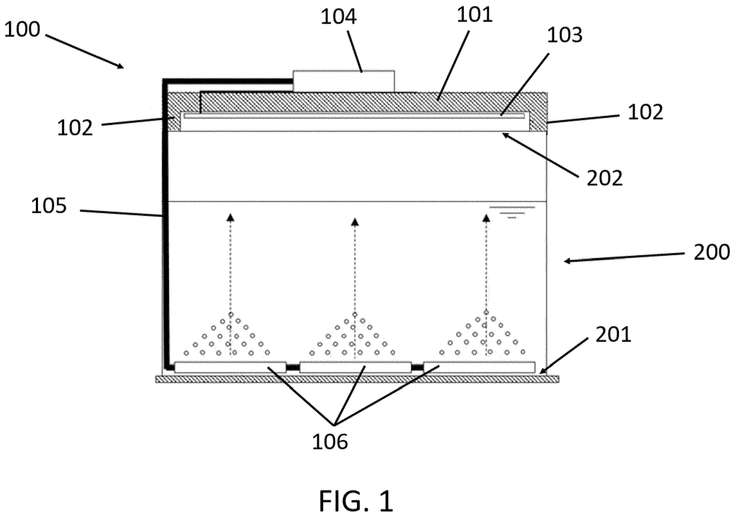

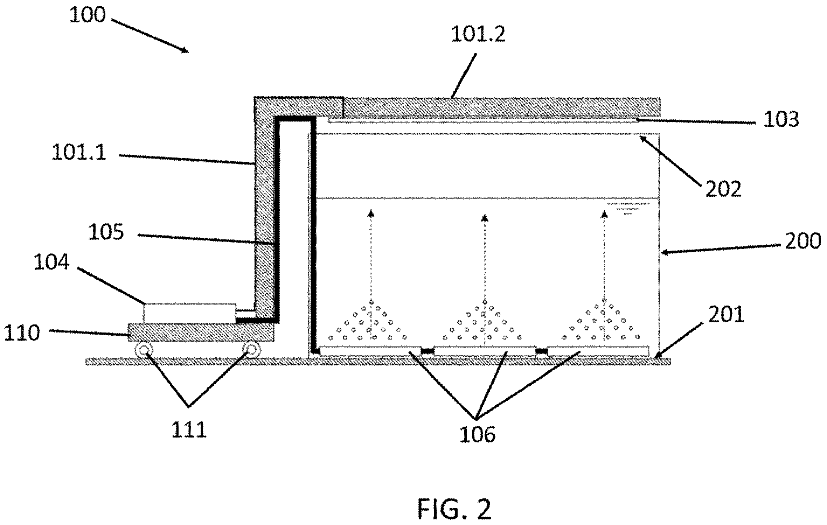

Figures 1 and 2 show schematic representations of systems (100) for the maintenance and sterilization of an emulsion housed within a reservoir (200) according to embodiments of the present invention.

In particular, a reservoir (200) can be seen, housing within it a cutting fluid intended for use in an industrial cutting and/or machining process, more specifically an oil emulsion. In an embodiment, said reservoir (200) has a rectangular structure and comprises a lower base (201) supported on a flat surface and, opposite said lower base (201), an opening (202). In other embodiments, the reservoir (200) may have a substantially cylindrical structure or another shape.

For the maintenance and sterilization of said oil emulsion housed inside the reservoir (200), a system (100) according to the invention is implemented and arranged in relation to said reservoir (200).

In particular, the operating principles of said system (100) are the following:
- the implementation of a UV light source illuminating the oil emulsion housed inside the reservoir (200), from a location close to the opening (202) thereof; and
- the generation of a turbulence within the oil emulsion to cause a recirculation thereof that, on the one hand, prevents the oil emulsion from demulsifying and, on the other hand, allows the UV light to act on the entire recirculated fluid, and not exclusively on the upper surface thereof when said fluid is at rest.

Specifically, the system (100) of the embodiment shown in Figure 1 comprises a supporting structure (101) configured to be arranged, at least partially, over the opening (202) of the reservoir (200), in an operating mode of the system (100). The supporting structure (101) comprises at least one coupling portion (102) configured to rest onto the contour of the opening (202) of the reservoir (200) such that, in an operating mode of the system, the UV lamp (103) illuminates the opening (202) of said reservoir (200). In the embodiment of Figure 1, the at least one coupling portion (102) is embodied as two flanges (102).

More in particular, as can be seen, the supporting structure (101) has a substantially rectangular cross-section, with dimensions that allow the supporting structure (101) to be arranged, by means of the two flanges (102) located on its periphery, directly on the contour of the opening of the reservoir shown in Figure 1.

In particular, as can be seen, in the embodiment of figure 1, the supporting structure (101) is located directly on top of the reservoir (200) by means of the two flanges (102), covering in its entirety the opening (202) of the reservoir (200). On the lower side of the supporting structure (101), facing downwards to illuminate the opening (202) of the reservoir (200), there is a UV lamp (103) which, as can be seen, has a laminar, plate-like shape covering most of the surface of the opening (202) of the reservoir (200).

In an embodiment, the supporting structure (101) comprises an extendable or telescopic section that allows increasing the relative dimension of the supporting structure (101), to allow the supporting structure (101), where the UV light sources are arranged, to rest onto the contour of the opening (202) of a reservoir (200), regardless of the dimensions of the reservoir (200).

In one embodiment, a plurality of UV lamps (103) are installed in the supporting structure (101) to be capable of illuminating an opening (202) of predefined size, each of said UV lamps (103) being controllable by a processing means (108) and occupying a portion of the total surface area of the portion of the supporting structure (101) on which they are installed. According to an embodiment, a subset of said UV lamps (103) may be activated depending on the size of the opening (202) of the reservoir (200) and/or the surface to be irradiated with UV light.

In an embodiment, the flanges (102) allow the supporting structure (101) to be arranged over the opening (202) of the reservoir (200) such that the portion of the supporting structure (101) on which the UV lamps (103) are implemented to be at a certain distance above said opening (202), so that the UV light sources bathe the upper fluid interface of the oil emulsion stored inside the reservoir (200) while direct contact between the UV lamps (103) and the oil emulsion is prevented.

Although a front cross-section of the system (100) and the reservoir (200) is shown, it will be understood that the elements shown schematically have volume and that said elements will extend orthogonally, outwardly and inwardly with respect to the view provided in Figure 1.

With respect to the configuration of the supporting structure shown in Figure 1, it is preferably made of metal and/or methacrylate, for example with a cross-sectional thickness of 10 mm.

With respect to the generation of turbulence within the oil emulsion to cause recirculation thereof, the system (100) comprises a conduit (105) configured for establishing a fluidic connection between a source of compressed air (104) and the interior of the reservoir (200). In particular, the system (100) shown in Figure 1 comprises a source (104) of compressed air and, more in particular, it can be seen that said conduit (105) extends from the source of compressed air (104) a distance along the upper side of the supporting structure (101), and then separates from supporting structure (101) and is inserted into the interior of the reservoir (200).

In particular, as can be seen in Figure 1, said conduit (105) reaches the lower base (201) of said reservoir (200), along which said conduit (105) extends, said conduit (105) comprising diffusion means (106) embodied as a plurality of porous blocks from which compressed air is provided in the form of bubbles, from the base (201) of the reservoir (200), through the oil emulsion, in an upward direction, which are schematically represented as three discontinuous pyramids formed of circumferences.

In other embodiments, the conduit (105) is configured to be connected to an external source (104) of compressed air, wherein the source of compressed air is not part of the system (100).

Figure 2 shows a schematic representation of a system (100) for the maintenance and sterilization of an emulsion housed within a reservoir (200) according to another embodiment of the present invention.

Specifically, the system (100) of the embodiment shown in Figure 2 comprises a supporting structure (101) comprising a main body (101.1) and a cantilever portion (101.2) projecting, substantially orthogonally, from said main body (101.1). In turn, the main body (101.1) of the supporting structure (101) projects substantially orthogonally from a pedestal portion (110) of the supporting structure (101), said pedestal portion (110) comprising wheels (111) on a lower surface thereof. The pedestal portion (110) is responsible for supporting and providing stability to the entire set of elements of the system (100) when supported on the ground or on a surface. For that purpose, said pedestal portion (110) is dimensioned in such a way as to prevent overturning of the system (100).

As can be seen, the relative height of the main body (101.1) with respect to the reservoir (200) is such as to allow the cantilever portion (101.2) to project at a sufficient distance with respect to the opening (202) of said reservoir (200), avoiding direct contact with the oil emulsion at all times.

In an embodiment, the supporting structure (101) (which comprises the main body (101.1) and the cantilever (101.2) portion on which the UV lamps (103) are implemented) is provided with suitable dimensions which permit said cantilever portion (101.2) to project over the opening (202) of the reservoir (200) so that the UV light sources bathe the upper fluid interface of the emulsion stored inside the reservoir (200).

In an embodiment, a UV lamp (103) is installed in the cantilever portion (101.2), said UV lamp (103) being controllable by a processing means (108) and occupying a portion of the total surface area of the side of the cantilever portion (101.2) on which it is installed.

In an embodiment, the main body (101.1) of the supporting structure (101) comprises an extendable or telescopic section that allows increasing the relative height of the main body (101.1), to allow the cantilever portion (101.2), where the UV light source is arranged, to be put at a certain distance above the opening (202) of a reservoir (200), regardless of the dimensions of the reservoir (200).

As can be seen, in the embodiment of figure 2, the cantilever portion (101.2) of the system (100) projects orthogonally from the main body (101.1) of the supporting structure (101), covering in its entirety the opening (202) of the reservoir (200). On the lower side of said cantilever portion (101.2), facing downwards to illuminate the opening (202) of the reservoir (200), there is a UV lamp (103) which, as can be seen, has a laminar, plate-like shape covering most of the surface of the opening (202) of the reservoir (200). In this sense, although a front cross-section of the system (100) and the reservoir (200) is shown, it will be understood that the elements shown schematically have volume and that said elements will extend orthogonally, outwardly and inwardly with respect to the view provided in Figure 2.

Alternatively or additionally to a plate-like shaped UV lamp, a plurality of UV lamps may be provided arranged to be capable of illuminating an opening (202) of predefined size. According to an embodiment, a subset of said UV lamps (103) may be activated depending on the size of the opening (202) of the reservoir (200) and/or the surface to be irradiated with UV light.

With respect to the configuration of the supporting structure (101) shown in Figure 2, the main body (101.1), the pedestal portion (110) and the cantilever portion (101.2) are preferably made of metal and/or methacrylate, for example with a cross-sectional thickness of 10 mm.

With respect to the generation of turbulence within the oil emulsion to cause recirculation thereof, the system (100) comprises a conduit (105) configured for establishing a fluidic connection between a source of compressed air (104) and the interior of the reservoir (200). In particular, the system (100) shown in Figure 2 comprises a source (104) of compressed air and, more in particular, it can be seen that said conduit (105) extends from the source of compressed air (104) a distance along the pedestal portion (110) of the supporting structure (101), and is coupled to the main body (101.1) along substantially its entire height, up to the point at which it encounters the cantilever portion (101.), and then engages said cantilever portion (101.2) along a portion of its length, and then separates from said cantilever portion (101.2) and is inserted into the interior of the reservoir (200).

In particular, as can be seen in Figure 2, said conduit (105) reaches the lower base (201) of said reservoir (200), along which said conduit (105) extends, said conduit (105) comprising diffusion means (106) embodied as a plurality of porous blocks from which compressed air is provided in the form of bubbles, from the base (201) of the reservoir (200), through the oil emulsion, in an upward direction, which are schematically represented as three discontinuous pyramids formed of circumferences.

In other embodiments, the conduit (105) is configured to be connected to an external source (104) of compressed air, wherein the source of compressed air is not part of the system (100).

The above description in relation to Figures 1 and 2 corresponds to an analysis of two respective embodiments of systems (100) according to the invention from a constructive approach, i.e., highlighting and drawing the attention to the hardware of the systems (100).

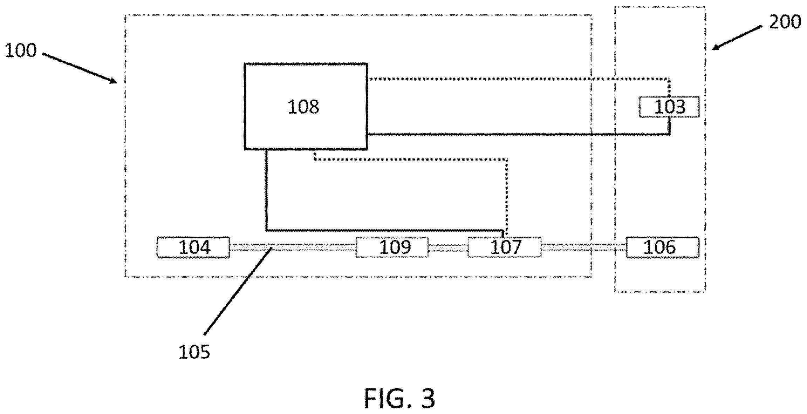

Figure 3 shows a block diagram depicting the manner in which the elements shown in any of Figures 1 and 2 relate to each other from a functional perspective, including for that purpose control and programmable electronic elements.

In particular, the embodiment of the system (100) shown in Figures 1, 2 and 3 comprises a controller, or processing means (108), configured to control the operation of the system (100). Said processing means (108), as well as the corresponding electromechanical auxiliary elements, may be housed in a box or case, together with the source of compressed air (104) shown in Figures 1 and 2, and more particularly on top the supporting structure (101) as shown in Figure 1; or on the pedestal portion (110) of the supporting structure (101) of the system (100) according to the embodiment shown in Figure 2.

With respect to the function of radiating the oil emulsion with UV light, in an embodiment the processing means (108) are configured for turning on/off the UV lamp (103) installed in the supporting structure (101).

As to the function of providing compressed air to the oil emulsion and generating turbulence in the form of bubbles provided from the base (201) of the reservoir (200) by the plurality of porous blocks (106) located at respective consecutive sections arranged at respective outlets of the conduit (105), the system (100) comprises a valve (107) configured to control the passage of compressed air through the conduit (105). Said valve is controlled by the processing means (108) of the system (100).

To exercise control over said elements, that is, to control the on/off of the UV lamp (103), and the supply of compressed air to the reservoir (200) through the valve (107) that regulates the passage of air through the conduit (105), the processing means (108) may be connected to additional elements and/or programmed with different modes of operation.

In an embodiment, the system (100) additionally comprises a pressure sensor (109) configured to determine the pressure of the compressed air in a section of the conduit (105) and to generate an electrical signal comprising information about the determined pressure.

Then, said electrical signal is sent to the processing means (108), which control the operation of the valve (107), which in the embodiment shown is a solenoid valve (107), based on the value determined by the pressure sensor (109) to maintain the pressure of the compressed air along the conduit (105) within a predetermined range. Preferably, the pressure of the compressed air along the conduit (105) is maintained within the range of 4 - 8 bar, and more particularly, within the range of 4 - 6 bar.

Also, the processing means (108) can be provided with different programs or modes of operation which make it possible to automate the operation of the system (100) and ensure the sustained maintenance over time of the optimum conditions of the oil emulsion, even without the presence or intervention of operators.

According to an embodiment of the invention, the processing means (108) are programmed to activate turbulence generation and UV exposure at interleaved intervals, that is, the periods in which the UV lamps (103) are active are alternated with the periods when compressed air is supplied, but are not allowed to occur simultaneously. According to this configuration of periods of activity of the two interleaved functionalities, the processing means (108) are programmed with a timer function for performing the following steps:
a) turning on the UV lamp (103) and controlling the valve (107) to prevent the passage of compressed air through the conduit (105) into the reservoir (200) until a predetermined time interval t₁ has elapsed; and
b) turning off the UV lamp (103) and controlling the valve (107) to allow the passage of compressed air through the conduit (105) into the reservoir (200) until a predetermined time interval t₂ has elapsed;
and wherein the processing means (108) are further programmed to perform step a) and step b) intermittently.

According to a different embodiment of the invention, the processing means (108) are programmed to activate turbulence generation and UV exposure at intermittent intervals, that is, periods of combined operation of both UV radiation and compressed air supply are alternated with periods of time during which both subsystems cease to operate. According to this configuration of intermittent intervals of operation of the system (100), the processing means (108) are programmed with a timer function for performing the following steps:
c) turning on the UV lamp (103) and controlling the valve (107) to allow the passage of compressed air through the conduit (105) into the reservoir (200) until a predetermined time interval t₃ has elapsed; and
d) turning off the UV lamp (103) and controlling the valve (107) to prevent the passage of compressed air through the conduit (105) into the reservoir (200) until a predetermined time interval t₄ has elapsed;
and wherein the processing means (108) are further programmed to perform step c) and step d) intermittently.

According to embodiments of the invention, the processing means (108) are programmed so as to implement different operating patterns of turbulence generation and UV radiation. That is, during an operating cycle, the system (100) may be operating for a predetermined time to activate turbulence generation and UV exposure at intermittent intervals, and, thereafter, switch the operation mode to activate turbulence generation and UV exposure at interleaved intervals; or vice versa.

On the other hand, the operating cycle of the system (100) may be desired to be stopped automatically after a certain elapsed operating time, for example, operating on the basis of the different programs/modes of operation described above.

For this purpose, in an embodiment the processing means (108) are programmed with a timer function for performing the following instruction:
e) turning off the UV lamp (103) and controlling the solenoid valve (107) to prevent the passage of compressed air through the conduit (105) into the reservoir (200) when a predetermined time interval t₅, during which the system (100) has been operating, has elapsed.

## Claims

1. System (100) for the maintenance and sterilization of oil emulsions housed within a reservoir (200), said reservoir (200) comprising a lower base (201) according to the direction of gravity and an opening (202) opposite said lower base (201), the system (100) comprising:
- a supporting structure (101) configured to be arranged, at least partially, in an operating mode of the system (100), over the opening (202) of said reservoir (200);
- at least one UV lamp (103) arranged on the supporting structure (101);
said system (100) being **characterized by** further comprising:
- at least one conduit (105) configured to be connected to a source (104) of compressed air and configured to be inserted, at least partially, in an operating mode of the system (100), inside said reservoir (200); and
- at least one compressed air diffusion means (106) provided in the conduit (105);
wherein said conduit (105) is configured to establish fluidic communication between the source (104) of compressed air and the interior of said reservoir (200) through the at least one compressed air diffusion means (106);
**characterised in that** the compressed air diffusion means (106) comprise at least one porous block adapted to be arranged at the lower base (201) of said reservoir (200); and
wherein the supporting structure (101) comprises at least one coupling portion (102) configured to rest onto the contour of the opening (202) of said reservoir (200) such that, in an operating mode of the system (100), the at least one UV lamp (103) illuminates the opening (202) of said reservoir (200);
wherein said at least one UV lamp (103) is decoupled from the interior of said reservoir (200);
wherein said at least one conduit (105) and said at least one compressed air diffusion means (106) are decoupled from said reservoir (200), allowing an easy introduction into, and extraction from, the interior of the reservoir which stores the oil emulsion.

2. The system (100) according to claim 1, wherein the supporting structure (101) comprises an extendable or telescoping portion which allows increasing a dimension of the supporting structure (101).

3. The system (100) according to any of the previous claims, wherein the at least one coupling portion (102) is embodied as two flanges (102).

4. System (100) for the maintenance and sterilization of oil emulsions housed within a reservoir (200), said reservoir (200) comprising a lower base (201) according to the direction of gravity and an opening (202) opposite said lower base (201), the system (100) comprising:
- a supporting structure (101) configured to be arranged, at least partially, in an operating mode of the system (100), over the opening (202) of said reservoir (200);
- at least one UV lamp (103) arranged on the supporting structure (101);
said system (100) being **characterized by** further comprising:
- at least one conduit (105) configured to be connected to a source (104) of compressed air and configured to be inserted, at least partially, in an operating mode of the system (100), inside said reservoir (200); and
- at least one compressed air diffusion means (106) provided in the conduit (105);
wherein said conduit (105) is configured to establish fluidic communication between the source (104) of compressed air and the interior of said reservoir (200) through the at least one compressed air diffusion means (106);
**characterised in that** the compressed air diffusion means (106) comprise at least one porous block adapted to be arranged at the lower base (201) of said reservoir (200); and
wherein the supporting structure (101) comprises a main body (101.1) and a cantilever portion (101.2) projecting from the main body (101.1), said cantilever portion (101.2) being configured to be arranged, at least partially, in an operating mode of the system (100), over the opening (202) of said reservoir (200), and wherein at least one UV lamp (103) is arranged on a lower side of the cantilever portion (101.2) facing downwards such that, in an operating mode of the system (100), the UV lamp (103) illuminates the opening (202) of said reservoir (200);
wherein said at least one UV lamp (103) is decoupled from the interior of said reservoir (200);
wherein said at least one conduit (105) and said at least one compressed air diffusion means (106) are decoupled from said reservoir (200), allowing an easy introduction into, and extraction from, the interior of the reservoir which stores the oil emulsion.

5. The system (100) according to claim 4, wherein the main body (101.1) and/or the cantilever portion (101.2) of the supporting structure comprises at least one extendable or telescoping portion.

6. The system (100) according to any of claims 4 or 5, wherein the supporting structure comprises a pedestal portion (110) arranged on a lowermost portion of the main body (101.1), said pedestal portion (110) comprising a substantially flat lower side, and being provided with a shape configured for withstanding the overturning moment caused by the main body (101.1) and the cantilever portion (101.2) of the supporting structure.

7. The (100) system according to any of claims 4 to 6, wherein the main body (101.1) of the supporting structure projects substantially perpendicular from the pedestal portion (110), and/or wherein the cantilever portion (101.2) projects essentially perpendicular from the main body (101.1).

8. The system (100) according to any of the previous claims, further comprising processing means (108) configured for turning on/off the UV lamp (103).

9. The system (100) according to claim 8, further comprising a valve (107) configured to control the passage of compressed air through the conduit (105); wherein the processing means (108) are configured for controlling the operation of the valve (107).

10. The system (100) according to claim 9, further comprising a pressure sensor (109) configured to:
- determine the pressure of the compressed air in a section of the conduit (105);
- generate an electrical signal comprising information about the determined pressure; and
- send said electrical signal to the processing means (108),
wherein the processing means (108) are further configured to control the operation of the valve (107) based on the value determined by the pressure sensor (109) to maintain the pressure of the compressed air along the conduit (105) within a predetermined range.

11. The system (100) according to claim 10, wherein the processing means (108) are configured to control the operation of the valve (107), based on the value of the pressure determined by the pressure sensor (109), to maintain the pressure of the compressed air along the conduit (105) within the range of 4 - 8 bar.

12. The system (100) according to any of claims 9 to 11, wherein the processing means (108) are programmed with a timer function for performing the following steps:
a) turning on the UV lamp (103) and controlling the valve (107) to prevent the passage of compressed air through the conduit (105) into the reservoir (200) until a predetermined time interval t₁ has elapsed; and
b) turning off the UV lamp (103) and controlling the valve (107) to allow the passage of compressed air through the conduit (105) into the reservoir (200) until a predetermined time interval t₂ has elapsed;
and wherein the processing means (108) are further programmed to perform step a) and step b) intermittently.

13. **The** system (100) according to any of claims 9 to 12, wherein the processing means (108) are programmed with a timer function for performing the following steps:
c) turning on the UV lamp (103) and controlling the valve (107) to allow the passage of compressed air through the conduit (105) into the reservoir (200) until a predetermined time interval t₃ has elapsed; and
d) turning off the UV lamp (103) and controlling the valve (107) to prevent the passage of compressed air through the conduit (105) into the reservoir (200) until a predetermined time interval t₄ has elapsed;
and wherein the processing means (108) are further programmed to perform step c) and step d) intermittently.

14. The system (100) according to any of claims 9 to 13, wherein the processing means (108) are programmed with a timer function for performing the following step:
e) turning off the UV lamp (103) and controlling the valve (107) to prevent the passage of compressed air through the conduit (105) into the reservoir (200) when a predetermined time interval t₅, during which the system (100) has been operating, has elapsed.

15. The system (100) according to any of the previous claims, further comprising a source (104) of compressed air.

## Patentansprüche

1. System (100) zur Aufrechterhaltung und Sterilisation von Ölemulsionen, die in einem Behälter (200) aufgenommen sind, wobei der Behälter (200) eine bezüglich der Schwerkraftrichtung untere Basis (201) und eine der unteren Basis (201) gegenüberliegende Öffnung (202) umfasst, wobei das System (100) umfasst:
- eine Trägerstruktur (101), die dazu konfiguriert ist, in einem Betriebsmodus des Systems (100) zumindest teilweise über der Öffnung (202) des Behälters (200) angeordnet zu sein;
- mindestens eine UV-Lampe (103), die an der Trägerstruktur (101) angeordnet ist;
wobei das System (100) **dadurch gekennzeichnet ist, dass** es ferner umfasst:
- mindestens eine Leitung (105), die dazu konfiguriert ist, mit einer Druckluftquelle (104) verbunden zu werden, und die dazu konfiguriert ist, in einem Betriebsmodus des Systems (100) zumindest teilweise in den Behälter (200) eingeführt zu werden; und
- mindestens ein Druckluft-Diffusionsmittel (106), das in der Leitung (105) vorgesehen ist;
wobei die Leitung (105) dazu konfiguriert ist, über das mindestens eine Druckluft-Diffusionsmittel (106) eine Fluidverbindung zwischen der Druckluftquelle (104) und dem Inneren des Behälters (200) herzustellen;
**dadurch gekennzeichnet, dass** die Druckluft-Diffusionsmittel (106) mindestens einen porösen Block umfassen, der dazu angepasst ist, an der unteren Basis (201) des Behälters (200) angeordnet zu sein; und
wobei die Trägerstruktur (101) mindestens einen Kopplungsabschnitt (102) umfasst, der dazu konfiguriert ist, auf dem Rand der Öffnung (202) des Behälters (200) aufzuliegen, derart, dass in einem Betriebsmodus des Systems (100) die mindestens eine UV-Lampe (103) die Öffnung (202) des Behälters (200) beleuchtet;
wobei die mindestens eine UV-Lampe (103) vom Inneren des Behälters (200) entkoppelt ist;
wobei die mindestens eine Leitung (105) und das mindestens eine Druckluft-Diffusionsmittel (106) vom Behälter (200) entkoppelt sind, wodurch ein einfaches Einführen in das Innere und ein einfaches Herausführen aus dem Inneren des die Ölemulsion speichernden Behälters ermöglicht wird.

2. Das System (100) nach Anspruch 1, wobei die Trägerstruktur (101) einen ausziehbaren oder teleskopierbaren Abschnitt umfasst, der eine Vergrößerung einer Abmessung der Trägerstruktur (101) ermöglicht.

3. Das System (100) nach einem der vorhergehenden Ansprüche, wobei der mindestens eine Kopplungsabschnitt (102) als zwei Flansche (102) ausgebildet ist.

4. System (100) zur Aufrechterhaltung und Sterilisation von Ölemulsionen, die in einem Behälter (200) aufgenommen sind, wobei der Behälter (200) eine bezüglich der Schwerkraftrichtung untere Basis (201) und eine der unteren Basis (201) gegenüberliegende Öffnung (202) umfasst, wobei das System (100) umfasst:
- eine Trägerstruktur (101), die dazu konfiguriert ist, in einem Betriebsmodus des Systems (100) zumindest teilweise über der Öffnung (202) des Behälters (200) angeordnet zu sein;
- mindestens eine UV-Lampe (103), die an der Trägerstruktur (101) angeordnet ist;
wobei das System (100) **dadurch gekennzeichnet ist, dass** es ferner umfasst:
- mindestens eine Leitung (105), die dazu konfiguriert ist, mit einer Druckluftquelle (104) verbunden zu werden, und die dazu konfiguriert ist, in einem Betriebsmodus des Systems (100) zumindest teilweise in den Behälter (200) eingeführt zu werden; und
- mindestens ein Druckluft-Diffusionsmittel (106), das in der Leitung (105) vorgesehen ist;
wobei die Leitung (105) dazu konfiguriert ist, über das mindestens eine Druckluft-Diffusionsmittel (106) eine Fluidverbindung zwischen der Druckluftquelle (104) und dem Inneren des Behälters (200) herzustellen;
**dadurch gekennzeichnet, dass** die Druckluft-Diffusionsmittel (106) mindestens einen porösen Block umfassen, der dazu angepasst ist, an der unteren Basis (201) des Behälters (200) angeordnet zu sein; und
wobei die Trägerstruktur (101) einen Hauptkörper (101.1) und einen von dem Hauptkörper (101.1) vorstehenden Auslegerabschnitt (101.2) umfasst, wobei der Auslegerabschnitt (101.2) dazu konfiguriert ist, in einem Betriebsmodus des Systems (100) zumindest teilweise über der Öffnung (202) des Behälters (200) angeordnet zu sein, und wobei mindestens eine UV-Lampe (103) an einer nach unten weisenden unteren Seite des Auslegerabschnitts (101.2) angeordnet ist, derart, dass in einem Betriebsmodus des Systems (100) die UV-Lampe (103) die Öffnung (202) des Behälters (200) beleuchtet;
wobei die mindestens eine UV-Lampe (103) vom Inneren des Behälters (200) entkoppelt ist;
wobei die mindestens eine Leitung (105) und das mindestens eine Druckluft-Diffusionsmittel (106) vom Behälter (200) entkoppelt sind, wodurch ein einfaches Einführen in das Innere und ein einfaches Herausführen aus dem Inneren des die Ölemulsion speichernden Behälters ermöglicht wird.

5. Das System (100) nach Anspruch 4, wobei der Hauptkörper (101.1) und/oder der Auslegerabschnitt (101.2) der Trägerstruktur mindestens einen ausziehbaren oder teleskopierbaren Abschnitt umfasst.

6. Das System (100) nach einem der Ansprüche 4 oder 5, wobei die Trägerstruktur einen Sockelabschnitt (110) umfasst, der an einem untersten Abschnitt des Hauptkörpers (101.1) angeordnet ist, wobei der Sockelabschnitt (110) eine im Wesentlichen flache Unterseite umfasst und mit einer Form versehen ist, die dazu konfiguriert ist, dem durch den Hauptkörper (101.1) und den Auslegerabschnitt (101.2) der Trägerstruktur verursachten Kippmoment standzuhalten.

7. Das (100) System nach einem der Ansprüche 4 bis 6, wobei der Hauptkörper (101.1) der Trägerstruktur im Wesentlichen senkrecht von dem Sockelabschnitt (110) vorsteht, und/oder wobei der Auslegerabschnitt (101.2) im Wesentlichen senkrecht von dem Hauptkörper (101.1) vorsteht.

8. Das System (100) nach einem der vorhergehenden Ansprüche, ferner umfassend Verarbeitungsmittel (108), die dazu konfiguriert sind, die UV-Lampe (103) ein-/auszuschalten.

9. Das System (100) nach Anspruch 8, ferner umfassend ein Ventil (107), das dazu konfiguriert ist, den Durchtritt von Druckluft durch die Leitung (105) zu steuern; wobei die Verarbeitungsmittel (108) dazu konfiguriert sind, den Betrieb des Ventils (107) zu steuern.

10. Das System (100) nach Anspruch 9, ferner umfassend einen Drucksensor (109), der dazu konfiguriert ist:
- den Druck der Druckluft in einem Abschnitt der Leitung (105) zu bestimmen;
- ein elektrisches Signal zu erzeugen, das Informationen über den bestimmten Druck umfasst; und
- das elektrische Signal an die Verarbeitungsmittel (108) zu senden,
wobei die Verarbeitungsmittel (108) ferner dazu konfiguriert sind, den Betrieb des Ventils (107) basierend auf dem durch den Drucksensor (109) bestimmten Wert zu steuern, um den Druck der Druckluft entlang der Leitung (105) innerhalb eines vorbestimmten Bereichs zu halten.

11. Das System (100) nach Anspruch 10, wobei die Verarbeitungsmittel (108) dazu konfiguriert sind, den Betrieb des Ventils (107) basierend auf dem Wert des durch den Drucksensor (109) bestimmten Drucks zu steuern, um den Druck der Druckluft entlang der Leitung (105) innerhalb des Bereichs von 4-8 bar zu halten.

12. Das System (100) nach einem der Ansprüche 9 bis 11, wobei die Verarbeitungsmittel (108) mit einer Zeitgeberfunktion programmiert sind, um die folgenden Schritte auszuführen:
a) Einschalten der UV-Lampe (103) und Steuern des Ventils (107), um den Durchtritt von Druckluft durch die Leitung (105) in den Behälter (200) zu verhindern, bis ein vorbestimmtes Zeitintervall t₁ verstrichen ist; und
b) Ausschalten der UV-Lampe (103) und Steuern des Ventils (107), um den Durchtritt von Druckluft durch die Leitung (105) in den Behälter (200) zuzulassen, bis ein vorbestimmtes Zeitintervall t₂ verstrichen ist;
und wobei die Verarbeitungsmittel (108) ferner dazu programmiert sind, Schritt a) und Schritt b) intermittierend auszuführen.

13. Das System (100) nach einem der Ansprüche 9 bis 12, wobei die Verarbeitungsmittel (108) mit einer Zeitgeberfunktion programmiert sind, um die folgenden Schritte auszuführen:
c) Einschalten der UV-Lampe (103) und Steuern des Ventils (107), um den Durchtritt von Druckluft durch die Leitung (105) in den Behälter (200) zuzulassen, bis ein vorbestimmtes Zeitintervall t₃ verstrichen ist; und
d) Ausschalten der UV-Lampe (103) und Steuern des Ventils (107), um den Durchtritt von Druckluft durch die Leitung (105) in den Behälter (200) zu verhindern, bis ein vorbestimmtes Zeitintervall t₄ verstrichen ist;
und wobei die Verarbeitungsmittel (108) ferner dazu programmiert sind, Schritt c) und Schritt d) intermittierend auszuführen.

14. Das System (100) nach einem der Ansprüche 9 bis 13, wobei die Verarbeitungsmittel (108) mit einer Zeitgeberfunktion programmiert sind, um den folgenden Schritt auszuführen:
e) Ausschalten der UV-Lampe (103) und Steuern des Ventils (107), um den Durchtritt von Druckluft durch die Leitung (105) in den Behälter (200) zu verhindern, wenn ein vorbestimmtes Zeitintervall t₅, während dessen das System (100) in Betrieb gewesen ist, verstrichen ist.

15. Das System (100) nach einem der vorhergehenden Ansprüche, ferner umfassend eine Druckluftquelle (104).

## Revendications

1. Système (100) pour l'entretien et la stérilisation d'émulsions d'huile contenues dans un réservoir (200), ledit réservoir (200) comprenant une base inférieure (201) par rapport à la direction de la gravité et une ouverture (202) opposée à ladite base inférieure (201), le système (100) comprenant :
- une structure de support (101) configurée pour être disposée, au moins partiellement, dans un mode de fonctionnement du système (100), au-dessus de l'ouverture (202) dudit réservoir (200) ;
- au moins une lampe UV (103) disposée sur la structure de support (101) ;
ledit système (100) étant **caractérisé en ce qu'**il comprend en outre :
- au moins un conduit (105) configuré pour être connecté à une source (104) d'air comprimé et configuré pour être inséré, au moins partiellement, dans un mode de fonctionnement du système (100), à l'intérieur dudit réservoir (200) ; et
- au moins un moyen de diffusion d'air comprimé (106) prévu dans le conduit (105) ;
dans lequel ledit conduit (105) est configuré pour établir une communication fluidique entre la source (104) d'air comprimé et l'intérieur dudit réservoir (200) par l'intermédiaire dudit au moins un moyen de diffusion d'air comprimé (106) ;
**caractérisé en ce que** les moyens de diffusion d'air comprimé (106) comprennent au moins un bloc poreux adapté pour être disposé à la base inférieure (201) dudit réservoir (200) ; et
dans lequel la structure de support (101) comprend au moins une partie d'accouplement (102) configurée pour reposer sur le contour de l'ouverture (202) dudit réservoir (200) de telle sorte que, dans un mode de fonctionnement du système (100), la au moins une lampe UV (103) éclaire l'ouverture (202) dudit réservoir (200) ;
dans lequel ladite au moins une lampe UV (103) est découplée de l'intérieur dudit réservoir (200) ;
dans lequel ledit au moins un conduit (105) et ledit au moins un moyen de diffusion d'air comprimé (106) sont découplés dudit réservoir (200), permettant une introduction facile dans, et une extraction hors de, l'intérieur du réservoir qui stocke l'émulsion d'huile.

2. Le système (100) selon la revendication 1, dans lequel la structure de support (101) comprend une partie extensible ou télescopique qui permet d'augmenter une dimension de la structure de support (101).

3. Le système (100) selon l'une quelconque des revendications précédentes, dans lequel la au moins une partie d'accouplement (102) est réalisée sous la forme de deux brides (102).

4. Système (100) pour l'entretien et la stérilisation d'émulsions d'huile contenues dans un réservoir (200), ledit réservoir (200) comprenant une base inférieure (201) par rapport à la direction de la gravité et une ouverture (202) opposée à ladite base inférieure (201), le système (100) comprenant :
- une structure de support (101) configurée pour être disposée, au moins partiellement, dans un mode de fonctionnement du système (100), au-dessus de l'ouverture (202) dudit réservoir (200) ;
- au moins une lampe UV (103) disposée sur la structure de support (101) ;
ledit système (100) étant **caractérisé en ce qu'**il comprend en outre :
- au moins un conduit (105) configuré pour être connecté à une source (104) d'air comprimé et configuré pour être inséré, au moins partiellement, dans un mode de fonctionnement du système (100), à l'intérieur dudit réservoir (200) ; et
- au moins un moyen de diffusion d'air comprimé (106) prévu dans le conduit (105) ;
dans lequel ledit conduit (105) est configuré pour établir une communication fluidique entre la source (104) d'air comprimé et l'intérieur dudit réservoir (200) par l'intermédiaire dudit au moins un moyen de diffusion d'air comprimé (106) ;
**caractérisé en ce que** les moyens de diffusion d'air comprimé (106) comprennent au moins un bloc poreux adapté pour être disposé à la base inférieure (201) dudit réservoir (200) ; et
dans lequel la structure de support (101) comprend un corps principal (101.1) et une partie en porte-à-faux (101.2) en saillie par rapport au corps principal (101.1), ladite partie en porte-à-faux (101.2) étant configurée pour être disposée, au moins partiellement, dans un mode de fonctionnement du système (100), au-dessus de l'ouverture (202) dudit réservoir (200), et dans lequel au moins une lampe UV (103) est disposée sur un côté inférieur de la partie en porte-à-faux (101.2) tournée vers le bas de telle sorte que, dans un mode de fonctionnement du système (100), la lampe UV (103) éclaire l'ouverture (202) dudit réservoir (200) ;
dans lequel ladite au moins une lampe UV (103) est découplée de l'intérieur dudit réservoir (200) ;
dans lequel ledit au moins un conduit (105) et ledit au moins un moyen de diffusion d'air comprimé (106) sont découplés dudit réservoir (200), permettant une introduction facile dans, et une extraction hors de, l'intérieur du réservoir qui stocke l'émulsion d'huile.

5. Le système (100) selon la revendication 4, dans lequel le corps principal (101.1) et/ou la partie en porte-à-faux (101.2) de la structure de support comprend au moins une partie extensible ou télescopique.

6. Le système (100) selon l'une quelconque des revendications 4 ou 5, dans lequel la structure de support comprend une partie de piédestal (110) disposée sur une partie la plus basse du corps principal (101.1), ladite partie de piédestal (110) comprenant une face inférieure sensiblement plane, et étant dotée d'une forme configurée pour résister au moment de renversement provoqué par le corps principal (101.1) et la partie en porte-à-faux (101.2) de la structure de support.

7. Le (100) système selon l'une quelconque des revendications 4 à 6, dans lequel le corps principal (101.1) de la structure de support se projette de manière sensiblement perpendiculaire à partir de la partie de piédestal (110), et/ou dans lequel la partie en porte-à-faux (101.2) se projette de manière essentiellement perpendiculaire à partir du corps principal (101.1).

8. Le système (100) selon l'une quelconque des revendications précédentes, comprenant en outre des moyens de traitement (108) configurés pour allumer/éteindre la lampe UV (103).

9. Le système (100) selon la revendication 8, comprenant en outre une vanne (107) configurée pour contrôler le passage d'air comprimé à travers le conduit (105) ; dans lequel les moyens de traitement (108) sont configurés pour commander le fonctionnement de la vanne (107).

10. Le système (100) selon la revendication 9, comprenant en outre un capteur de pression (109) configuré pour :
- déterminer la pression de l'air comprimé dans une section du conduit (105) ;
- générer un signal électrique comprenant des informations sur la pression déterminée ; et
- envoyer ledit signal électrique aux moyens de traitement (108),
dans lequel les moyens de traitement (108) sont en outre configurés pour commander le fonctionnement de la vanne (107) sur la base de la valeur déterminée par le capteur de pression (109) afin de maintenir la pression de l'air comprimé le long du conduit (105) dans une plage prédéterminée.

11. Le système (100) selon la revendication 10, dans lequel les moyens de traitement (108) sont configurés pour commander le fonctionnement de la vanne (107), en fonction de la valeur de la pression déterminée par le capteur de pression (109), afin de maintenir la pression de l'air comprimé le long du conduit (105) dans la plage de 4-8 bars.

12. Le système (100) selon l'une quelconque des revendications 9 à 11, dans lequel les moyens de traitement (108) sont programmés avec une fonction de minuterie pour effectuer les étapes suivantes :
a) allumer la lampe UV (103) et commander la vanne (107) pour empêcher le passage d'air comprimé à travers le conduit (105) dans le réservoir (200) jusqu'à ce qu'un intervalle de temps prédéterminé t₁ se soit écoulé ; et
b) éteindre la lampe UV (103) et commander la vanne (107) pour permettre le passage de l'air comprimé à travers le conduit (105) dans le réservoir (200) jusqu'à ce qu'un intervalle de temps prédéterminé t₂ se soit écoulé ;
et dans lequel les moyens de traitement (108) sont en outre programmés pour exécuter l'étape a) et l'étape b) de manière intermittente.

13. Le système (100) selon l'une quelconque des revendications 9 à 12, dans lequel les moyens de traitement (108) sont programmés avec une fonction de minuterie pour exécuter les étapes suivantes :
c) allumer la lampe UV (103) et commander la vanne (107) pour permettre le passage de l'air comprimé à travers le conduit (105) vers le réservoir (200) jusqu'à ce qu'un intervalle de temps prédéterminé t₃ se soit écoulé ; et
d) éteindre la lampe UV (103) et commander la vanne (107) pour empêcher le passage de l'air comprimé à travers le conduit (105) vers le réservoir (200) jusqu'à ce qu'un intervalle de temps prédéterminé t₄ se soit écoulé ;
et dans lequel les moyens de traitement (108) sont en outre programmés pour exécuter les étapes c) et d) de manière intermittente.

14. Le système (100) selon l'une quelconque des revendications 9 à 13, dans lequel les moyens de traitement (108) sont programmés avec une fonction de minuterie pour effectuer l'étape suivante :
e) éteindre la lampe UV (103) et commander la vanne (107) pour empêcher le passage de l'air comprimé à travers le conduit (105) vers le réservoir (200) lorsqu'un intervalle de temps prédéterminé t₅, pendant lequel le système (100) a fonctionné, s'est écoulé.

15. Le système (100) selon l'une quelconque des revendications précédentes, comprenant en outre une source (104) d'air comprimé.
